# EUROPÄISCHE PATENTSCHRIFT

(11) **EP 0 488 044 B1**
(45) Veröffentlichungstag und Bekanntmachung des Hinweises auf die Patenterteilung: **14.02.1996**
(21) Anmeldenummer: 91119823.2
(22) Anmeldetag: 21.11.1991
(51) Int. Cl.: A61K 31/55

(54) **Ein Imidazobenzodiazepin zur Behandlung von Schlafstörungen**
An imidazobenzodiazepin for treating sleep disorders
Une imidazobenzodiazepine pour le traitement des troubles du sommeil

(30) Priorität: 29.11.1990 CH 3776/90
(43) Veröffentlichungstag der Anmeldung: 03.06.1992
(73) Patentinhaber: F. HOFFMANN-LA ROCHE AG, CH-4002 Basel (CH)
(72) Erfinder: Amrein, Roman, CH-4126 Bettingen (CH); Laurent, Jean-Paul, F-68100 Mulhouse (FR)
(74) Vertreter: Bertschinger, Christoph, Dr.

(56) Entgegenhaltungen:
- EP-A- 0 059 391
- EP-A- 0 331 871
- WO-A-90/02737
- US-A- 4 861 772
- INT. J. CLIN. PHARMACOL. THER. TOXICOL., Band 27, Nr. 2, Februar 1989, Seiten 51-65, DE; B. SALETU et al.: "On the central effects of a new partial benzodiazepine agonist RO 16-6028 in man: pharmaco-EEG and psychometric studies"
- DRUGS OF THE FUTURE, Band 13, Nr. 8, August 1988, Seiten 730-735; "RO 16-6028"
- PSYCHOPHARMACOLOGY, Band 97, Nr. 3, 1989, Seiten 388-391; C. BELZUNG et al.: "Behavioural effects of the benzodiazepine receptor partial agonist RO 16-6028 in mice"
- SOCIETY FOR NEUROSCIENCE ABSTRACTS, Band 15, Nr. 2, 1989, Seite 1234, Zusammenfassung Nr. 485.12; P.J.K.D. SCHREUR et al.: "The late exploratory test for hypnotics in mice"
- "The Merck Manual", 15. Ausgabe, 1987, Seiten 1375-1378, herausgeben von R. Berkow et al., Merck & Co., Inc., Rahway, NJ, US

## Beschreibung

Die vorliegende Erfindung betrifft die Verwendung von Bretazenil, t-Butyl (S)-8-brom-11,12,13,13a-tetrahydro-9-oxo-9H-imidazo[1,5-a]pyrrolo[2,1-c][1,4]benzodiazepin-1-carboxylat, der Formel 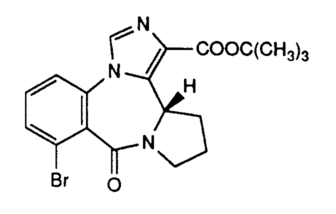
bei der Behandlung von Schlafstörungen. Gegenstand der vorliegenden Erfindung sind ferner die Verwendung von Bretazenil zur Herstellung von Mitteln zur Behandlung von Schlafstörungen und ein Verfahren und Mittel zur Behandlung von Schlafstörungen.

Bretazenil ist eine bekannte Substanz. Ihre Herstellung wird beispielsweise in der Europäischen Patentpublikation Nr. 59 391 beschrieben. In dieser Publikation werden auch die antikonvulsiven und anxiolytischen Eigenschaften dieser Verbindung beschrieben.

Bretazenil hat eine hohe Affinität zum Benzodiazepinrezeptor (BZR), dem modulatorischen Teil des Rezeptors für die Aminosäure GABA. GABA ist ein hemmender Botenstoff (Neurotransmitter) bestimmter Nervenzellen (Neuronen) des Gehirns. Die Ausschüttung von GABA durch einen Neuronentyp bewirkt Hemmung der Erregbarkeit von anderen Neuronen, was sich beispielsweise in einer anxiolytischen, antikonvulsiven,muskelrelaxierenden oder sedativ-hypnotischen Wirkung äussern kann. Es wurden drei Haupttypen von Stoffen gefunden, die an den BZR binden und als Liganden bezeichnet werden: (1) Die Agonisten, die die Hemmung durch GABA verstärken; (2) die sogenannten inversen Agonisten, die die Wirkung von GABA abschwächen; und (3) die Antagonisten, die die Wirkung von GABA nicht beeinflussen, aber deren Verstärkung oder Abschwächung durch Agonisten bzw. inverse Agonisten am BZR verhindern (s. Haefely, W., Handbook of Anxiety 3: 165-188, 1990). Wirksame Substanzen aus den drei Gruppen von Liganden am BZR haben generell eine hohe Affinität zu diesem Rezeptor, unterscheiden sich aber durch die sogenannte relative intrinsische Wirksamkeit, d.h. die Fähigkeit, die Wirkung von GABA zu beeinflussen. Während reine Antagonisten den BZR besetzen, die Wirkung von GABA aber nicht beeinflussen, erzielen volle Agonisten oder inverse Agonisten am BZR maximale Verstärkung oder Abschwächung der hemmenden Wirkung von GABA. Zwischen den Extremen sind Substanzen mit unterschiedlichem Grad von intrinsischer Wirksamkeit denkbar und wurden auch gefunden. Sie verhalten sich selbst wie schwache Agonisten oder inverse Agonisten, schwächen aber die Wirkung der jeweiligen vollen Agonisten deutlich ab. Solche Substanzen sind somit partielle Agonisten oder partielle inverse Agonisten.

Es hat sich nun gezeigt, dass Bretazenil ein solcher partieller Agonist oder Partialagonist am BZR ist. In Tierversuchen wurde festgestellt, daß Bretazenil eine hohe Affinität zum BZR besitzt, daß mit der Substanz aber lediglich Wirkungen zu erzielen sind, die denjenigen niedriger Dosen von Benzodiazepinen (BZD), wie zum Beispiel Diazepam, entsprechen. Die charakteristischen Wirkungen und Nebenwirkungen höherer und hoher Dosen der BZD, wie sedierende Wirkung, Muskelrelaxierung, Ataxie und Amnesie, konnten in klassischen Tierversuchen für Bretazenil nicht oder nur in subtoxischen Dosen gezeigt werden.

Es wurde nun überraschenderweise gefunden, daß Bretazenil an gesunden, freiwilligen Männern und Frauen (Probanden) bereits in niedrigen Dosen Schlaf induzieren kann und daß der induzierte Schlaf weitgehend dem natürlichen Schlaf entspricht.

Diese Eigenschaft von Bretazenil konnte anhand des nachstehend beschriebenen Doppelblindversuches ermittelt werden:
Es wurden je sechs männliche und weibliche Probanden im Durchschnittsalter von 24 Jahren und mit einem Durchschnittsgewicht von 58 kg in die Studie miteinbezogen. Die Probanden wurden veranlasst 0.5, 1 oder 2 mg Bretazenil in Tablettenform nach 21 Uhr zu sich zu nehmen. Dies erfolgte, nachdem die Probanden an zwei vorausgehenden Abenden identische Placebotabletten eingenommen hatten. Die zweite Placebonacht diente als Referenz (Vorkontrolle). Am Abend des Tages nach Bretazenil wurde wiederum Placebo verabreicht (Nachkontrolle). Die Probanden verbrachten die vier Nächte einer Session in einem Schlaflabor. Ihre Hirnstromkurven (EEG) und Körperbewegungen wurden dabei auf Magnetband aufgezeichnet und danach mittels eines Computers ausgewertet. Diese Methode erlaubt die exakte Erfassung des Schlafprofils.

Die in dieser Studie ermittelten Resultate (vgl. Tabelle I) zeigen ganz deutlich, dass Bretazenil in allen geprüften Dosen wirksam war. Bretazenil verkürzte die Zeit zum Einschlafen (Schlaflatenz) drastisch und verlängerte den Gesamtschlaf pro Nacht indem es den Normalschlaf (non REM-Schlaf) verlängerte und die Anzahl intermittierender kurzer Wachphasen reduzierte. Ebenso reduzierte Bretazenil die Anzahl der Körperbewegungen im Schlaf. Somit bewirkt Bretazenil ein schnelleres Einschlafen und eine Verlängerung und Stabilisierung des Schlafes. Im Normalschlaf reduzierte Bretazenil die Tiefschlafstadien 3 und 4 nicht oder nur unwesentlich, verlängerte aber insgesamt die Dauer des mittleren Schlafstadiums 2. Dieses ist durch das Auftreten sogenannter Schlafspindeln, charakteristischer Muster im EEG, gekennzeichnet. Deren Anzahl wurde aber nicht verändert. Reduziert wurde jedoch die Anzahl der K-Komplexe, anderer charakteristischer Muster im EEG, im Stadium 2. Diese werden auf akustische Störsignale zurückgeführt, die das schlafende Hirn erreichen. Ihre Reduktion deutet somit auf eine Vertiefung des Schlafes hin. Der oberflächliche Schlaf des Stadiums 1 wurde von Bretazenil eher verkürzt.

Der Schlaf eines gesunden Menschen gliedert sich üblicherweise in fünf Schlafzyklen, wobei jeder Zyklus mit einer Traumphase beendet wird. Bretazenil veränderte die Anzahl der Zyklen nicht, verlängerte aber den ersten Zyklus und damit die Zeit bis zum Auftreten der ersten Traumphase (REM-Schlaflatenz). Der Traumschlaf, auch REM-Schlaf genannt, weil darin schnelle Augenbewegungen (rapid eye movements) auftreten, wurde aber insgesamt von Bretazenil nur unwesentlich verkürzt. Die Dichte der schnellen Augenbewegungen (REMs) im REM-Schlaf wurde vermindert.

**Tabelle I**

| Wirkungen von Bretazenil auf den Schlaf gesunder Probanden | | | |
|---|---|---|---|
| | Verabreichte Dosis in mg p.o. | | |
| | 0,5 | 1 | 2 |
| Schlaflatenz | 33* | 30* | 20* |
| Gesamtschlaf | + 45 min* | + 44 min* | + 45 min* |
| Intermittierende Wachphasen | 32* | 25* | 22* |
| Bewegungen | 80* | 74* | 69* |
| non REM-Schlaf | + 60 min* | + 60 min* | + 62 min* |
| Stadium 1 | 98 | 65* | 79 |
| Stadium 2 | 130* | 128* | 136* |
| Stadium 3 | 90 | 90 | 87 |
| Stadium 4 | 87* | 103 | 79* |
| Spindeln in Stadium 2 | 101 | 107 | 107 |
| K-Komplexe in Stadium 2 | 71* | 63* | 61* |
| Dauer des ersten Schlafzyklus | 154* | 189* | 198* |
| REM-Schlaflatenz | 165* | 206* | 217* |
| REM-Schlaf Dauer | 91 | 83 | 85 |
| REMs im REM-Schlaf | 84 | 49* | 31* |
| Alle Werte, außer Gesamtschlaf und non REM-Schlaf, sind in % und beziehen sich auf die Vorkontrolle. Für Gesamtschlaf und non REM-Schlaf ist die absolute Verlängerung in Minuten angegeben. | | | |

| | | | |
|---|---|---|---|
| * p < 0,05 | | | |

Im Rahmen der vorliegenden Erfindung kann Bretazenil in Form von peroral, rektal und parenteral verabreichbaren pharmazeutischen Präparaten verwendet werden. Beispiele derartiger Präparate sind Tabletten, Lacktabletten, Dragées, Hart- und Weichgelatinekapseln, Suppositorien, Lösungen, Emulsionen oder Suspensionen. Als Darreichungsformen werden peroral verabreichbare Formen, insbesondere Tabletten, bevorzugt.

Zur Herstellung von pharmazeutischen Präparaten wird Bretazenil mit pharmazeutisch inerten, anorganischen oder organischen Trägermaterialien verarbeitet. Als Trägermaterialien eignen sich für Tabletten, Lacktabletten, Dragées und Hartgelatinekapseln beispielsweise Milchzucker, Maisstärke oder Derivate davon, Talk, Stearinsäure oder deren Salze und dergleichen. Für Weichgelatinekapseln eignen sich beispielsweise pflanzliche Oele, Wachse, Fette, halbfeste und flüssige Polyole und dergleichen. Für Suppositorien eignen sich beispielsweise natürliche oder gehärtete Oele, Wachse, Fette, halbfeste und flüssige Polyole und dergleichen. Zur Herstellung von Lösungen, Emulsionen und Suspensionen eignen sich als Trägermaterialien beispielsweise Wasser, Polyole, Saccharose, Invertzucker, Glucose und dergleichen.

Die pharmazeutischen Präparate können daneben noch die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und/oder Antioxidantien enthalten.

Wie bereits erwähnt, kann Bretazenil bei der Behandlung von Schlafstörungen verwendet werden. Die Dosierung kann je nach Schwere der Schlafstörungen, Alter und Gewicht des Patienten variieren und ist natürlich in jedem einzelnen Fall den individuellen Gegebenheiten anzupassen. Im allgemeinen dürfte bei peroraler Verabreichung eine Dosis von etwa 0,25 bis etwa 5 mg angemessen sein.

Das nachfolgende Beispiel beschreibt eine für die praktische Anwendung der vorliegenden Erfindung geeignete Dosierungsform. Es soll den Umfang der vorliegenden Erfindung jedoch in keiner Weise beschränken:

### Beispiel (Tablette)

| | |
|---|---|
| Bretazenil | 0,5 mg |
| Milchzucker | 126,5 mg |
| Maisstärke | 54,0 mg |
| Polyvinylpyrrolidon | 8,0 mg |
| Natrium-Carboxymethylstärke | 10,0 mg |
| Magnesiumstearat | 1,0 mg |
| Tablettengewicht | 200,0 mg |

Bretazenil, der Milchzucker und die Maisstärke werden gemischt und mit einer wässrigen Lösung von Polyvinylpyrrolidon granuliert. Das getrocknete und gebrochene Granulat wird mit der Natrium-Carboxymethylstärke und dem Magnesiumstearat gemischt, worauf man zu Tabletten mit einem Gewicht von 200 mg verpresst.

## Patentansprüche

1. Verwendung von Bretazenil, t-Butyl (S)-8-brom-11,12,13,13a-tetrahydro-9-oxo-9H-imidazo[1,5-a]pyrrolo[2,1-c][1,4]benzodiazepin-1-carboxylat zur Herstellung eines Arzneimittels zur Behandlung von Schlafstörungen.

## Claims

1. The use of bretazenil, t-butyl (S)-8-bromo-11,12,13, 13a-tetrahydro-9-oxo-9H-imidazo[1,5a]pyrrolo[2,1-c][1,4]-benzodiazepine-1-carboxylate, for the manufacture of a medicament for the treatment of sleep disorders.

## Revendications

1. Utilisation du Bretazenil, (S)-8-bromo-11,12,13,13a-tétrahydro-9-oxo-9H-imidazo[1,5-a]pyrrolo[2,1-c][1,4]benzodiazépine-1-carboxylate de tert-butyle, pour la préparation d'un médicament prévu pour le traitement des troubles du sommeil.
